# EUROPEAN PATENT SPECIFICATION

(11) **EP 2 696 700 B1**
(45) Date of publication and mention of the grant of the patent: **21.03.2018**
(21) Application number: 12716556.1
(22) Date of filing: 13.04.2012
(51) Int. Cl.: A23K 10/38, A23K 10/12, A23K 20/163, C12P 7/08, C12P 7/14

(54) **HYDROLYSIS AND FERMENTATION PROCESS FOR ANIMAL FEED PRODUCTION**
HYDROLYSE- UND FERMENTATIONS VERFAHREN FÜR TIERFUTTER
PROCÉDÉ D'HYDROLYSE ET DE FERMENTATION POUR FOURRAGES

(30) Priority: 13.04.2011 GB 201106261
(43) Date of publication of application: 19.02.2014
(73) Proprietor: Ensus UK Limited, Yarm, TS15 9BW (GB)
(72) Inventor: LYWOOD, Warwick, Yarm Durham TS15 9BW (GB); PINKNEY, John, Yarm Durham TS15 9BW (GB); JAVED, Muhammad, Essex IG2 6YF (GB); EDWARDS, James, Cheshire CW8 4RE (GB)
(74) Representative: Boult Wade Tennant
(86) International application number: PCT/GB2012/050827
(87) International publication number: WO 2012/140444

(56) References cited:
- WO-A1-2007/056321
- WO-A1-2007/110606
- FR-A1- 2 949 645
- GB-A- 1 549 573
- US-A1- 2003 044 951
- US-A1- 2010 143 974
- US-A1- 2010 196 979
- AMARTEY, S. A., ET AL.: "FERMENTATION OF A WHEAT STRAW ACID HYDROLYSATE BY BACILLUS STEAROTHERMOPHILUS T-13 IN CONTINUOUS CULTURE WITH PARTIAL CELL RECYCLE", PROCESS BIOCHEMISTRY., vol. 34, 1999, pages 289-294, XP002677729, XXXX
- VELASQUEZ-ARREDONDO H I ET AL: "Ethanol production process from banana fruit and its lignocellulosic residues: Energy analysis", ENERGY, PERGAMON PRESS, OXFORD, GB, vol. 35, no. 7, 1 July 2010 (2010-07-01), pages 3081-3087, XP027065226, ISSN: 0360-5442, DOI: 10.1016/J.ENERGY.2010.03.052 [retrieved on 2010-05-29]
- PICCOLO C ET AL: "A techno-economic comparison between two technologies for bioethanol production from lignocellulose", BIOMASS AND BIOENERGY, PERGAMON, AMSTERDAM, NL, vol. 33, no. 3, 1 March 2009 (2009-03-01), pages 478-491, XP025947825, ISSN: 0961-9534, DOI: 10.1016/J.BIOMBIOE.2008.08.008 [retrieved on 2008-10-26]

## Description

### FIELD OF THE INVENTION

The invention relates to processes for making an animal feed product and to animal feed products. In particular, the invention relates to processes which rely upon hydrolysis of fermentation feedstocks or non-ethanol by-products of a fermentation process and optionally fermentation in order to improve the nutritional content of the resultant animal feed products.

### BACKGROUND TO THE INVENTION

Work has been done to develop micro-organisms, including thermophillic *Geobacillus* micro-organisms, to produce bioethanol from either mixed pentose (C5) and hexose (C6) sugars, or the C5 sugars on their own. WO 2007/110606 describes thermophilic microorganisms transformed with a gene encoding an NAD-linked formate dehydrogenase in order to maximise ethanol production. WO 2006/117536 and WO 02/29030 each describe thermophilic microorganisms carrying an inactivated lactate dehydrogenase gene.

Linde (Bioresource Technology 99 (2008) 6506 - 6511) investigated theoretical increases in ethanol yield by applying heat treatment followed by enzymatic hydrolysis to residual starch-free cellulose and hemi-cellulose fractions of slurries obtained from process streams in a starch-to-ethanol plant. The process slurries investigated were the flour, the slurry after saccarification of the starch, before fermentation, and after fermentation. An increase of 14% in ethanol yield compared with starch-only utilization could theoretically be achieved, assuming fermentation of the additional pentose and hexose sugars liberated. While cellulose hydrolysis produces glucose, which is easily fermented to ethanol, hemi-cellulose hydrolysis produces a large proportion of pentose (C5) sugars. Pentose sugars require pentose-fermenting yeast, not currently used in industrial processes. The process of Linde is performed solely with a view to maximising ethanol yields.

Cookman (Bioresource Technology 100 (2009) 2012 - 2017) investigated the feasibility of extracting oil and protein from distiller's grain (DG) to obtain a higher-valued protein-rich product. Protein extractions were based upon aqueous ethanol, alkaline-ethanol, and aqueous enzyme treatments. The carbohydrate left behind was intended for conversion to fermentable sugars. The recovered protein was not examined to determine its value.

Misailidis (Chemical Engineering Research and Design 87 (2009) 1239 - 1250) investigated the economic feasibility of co-producing an arabinoxylan (AX) product with ethanol from wheat. Three scenarios were modelled: conventional wheat-to-bioethanol production with DDGS as the principal co-product; bioethanol production with co-production of AX using conventional hammer milling and sieving to recover the bran for AX extraction; and the use of pearling technology to recover bran for AX extraction. Sending bran removed via pearling directly to DDGS was not economic.

Srinivasan (Bioresource Technology 100 (2009) 3548 - 3555) describes a laboratory scale sieving and air classification process for the removal of fibre from DDGS.

US 2010/0196979 describes use of spent brewers grain as a biomass source for the production of ethanol and other products such as livestock feed.

WO 2010/107944 relates to conversion of lignocellulosic material to fermentable sugars and to additional products produced therefrom such as animal feeds.

US 6,444,437 relates to a two-step process to convert rural biomass and other cellulosic materials to a protein rich animal feed supplement.

US 2003/0232109 describes a process for producing a highly digestible high-protein product from corn endosperm based upon use of a dehulling and degermination step at the front end.

WO 82/01483 relates to a process and apparatus for recovering organic and inorganic matter from waste material.

WO 2005/079190 relates to pre-treatment steps to solubilise starch and enhance enzymatic digestibility of cellulose in the fibre.

US 2010/0167367 describes processes for the recovery of ethanol from various cellulosic feedstock materials.

WO 2009/079183 relies upon use of cellulolytic fermentation of the non-ethanol byproduct of a fermentation process in order to enhance protein levels in the feed mixture.

FR 2949645 describes processes for recovering the by-products of distillation.

WO 2007/056321 describes a method of dewatering whole stillage.

### DESCRIPTION OF THE INVENTION

Many co-product animal feed materials, such as distillers dried grain and solubles (DDGS), wheat bran, corn fibre and sugar beet pulp, used for animal feed, are not used as efficiently as some other animal feed products, such as soy meal. The reasons for this are that they have:
- lower protein content,
- higher fibre content
- high levels of soluble non starch polysaccharides (NSPs)
- contain reducing sugars
The high fibre content reduces the protein digestibility and metabolisable energy in pigs and poultry. Higher levels of NSPs cause fermentation in the hind gut of pigs, which limits DDGS inclusion rates in pigs and poultry. Reducing sugars, such as glucose, maltose and arabinose in DDGS and bran cause degradation of lysine (an essential amino acid) due to the Maillard reaction during the DDGS or bran drying processes,

The inventors have devised a process which aims to control the extent of an additional fermentation step to ethanol (over and above that performed in the existing biethanol production process) in order to upgrade the animal feed quality of the co-product stream (arising from the existing bioethanol production process). The invention is defined in the claims. Thus, according to a first aspect the invention provides a method for production of an animal feed product, the method comprising, as part of an existing bioethanol production process:
a) partial hydrolysis of the non-ethanol by-product of a fermentation process performed on a fermentation feedstock, which partial hydrolysis converts non starch polysaccharides to soluble oligomers and monomers, wherein the non-ethanol by-product of a fermentation process performed on a fermentation feedstock is, or is derived from, the still bottoms or stillage from ethanol production
b) fermentation of the soluble oligomers and monomers in the partially hydrolysed feedstock or non-ethanol by-product to produce additional ethanol compared to that produced by the existing bioethanol production process
c) recovery of the non-ethanol by-product from the fermentation of step b) to produce an animal feed product with improved nutritional content.
The method may also be considered as a method for upgrading an animal feed product, specifically one co-produced in a fermentation process.

Also described herein is a method for production of an animal feed product, in particular an animal feed product with improved nutritional content, the method comprising:
a) partial hydrolysis of the non-ethanol by-product of a fermentation process performed on a fermentation feedstock, which partial hydrolysis converts non starch polysaccharides to soluble oligomers and monomers
b) recovery of the partially hydrolysed product from step a), to exclude the soluble oligomers and monomers, to produce an animal feed product, more specifically an animal feed product with improved nutritional content.

This process may be considered a subset of the more general process, which is performed directly on the non-ethanol by-product of a fermentation process performed on a fermentation feedstock. Thus, this method may be included in the "back end" of existing bioethanol production plants as a means of upgrading the animal feed product produced as a co-product of the fermentation process. Partial hydrolysis followed by recovery of the partially hydrolysed product, not including the released reducing sugars, improves the relative nutritional content of the animal feed product.

According to the invention, the released soluble oligomers and monomers are fermented to ethanol.. In alternative examples, rather than fermenting the soluble oligomers and monomers they can simply be separated from the remainder of the partially hydrolysed product. For example, the insoluble product may be separated by centrifugation to produce the solid animal feed product.

Any suitable fermentation feedstock may be employed in the methods of the invention. Many bioethanol plants exist in which a range of materials are fermented to produce ethanol. In specific embodiments, the fermentation feedstock comprises a hemi-cellulose containing material, in particular plant material. Suitable examples include corn, wheat, barley and sugar beet pulp. The invention may rely upon thermophilic microorganisms capable of fermenting such hemi-cellulosic sugars derived from plant materials. The feedstock may additionally or alternatively comprise cellulose containing material. Fermentation may thus be of pentose and/or hexose sugars.

The invention relies upon partial hydrolysis of the non-ethanol by-product of a fermentation process performed on a fermentation feedstock, which partial hydrolysis converts non starch polysaccharides to soluble oligomers and monomers, wherein the non-ethanol by-product of a fermentation process performed on a fermentation feedstock is, or is derived from, the still bottoms or stillage from ethanol production. The hydrolysis is partial, which represents an important balance to ensure ethanol yields are improved compared to these achieved without hydrolysis whilst permitting the animal feed product to be upgraded. Maximising ethanol yields by also maximising hydrolysis may be to the detriment of the quality of the animal feed product. Similarly, extensive hydrolysis may increase process costs to such an extent that it becomes uneconomic. Thus, in specific embodiments the partial hydrolysis comprises up to around 75%, 70%, 65%, 60%, 55%, 50%, 45%, 40%, 35%, 30%, 25%, 20%, 15% or around 10% hydrolysis of the non starch polysaccharides (found in the fermentation feedstock ethanol by-product of a fermentation process).

The partial hydrolysis may be performed by any suitable means. In specific embodiments the partial hydrolysis is performed chemically and/or enzymatically. Typically, if chemical hydrolysis is utilised the fermentation will need to be performed separately, under different reaction conditions. In certain embodiments, particularly where enzymatic hydrolysis is performed, the partial hydrolysis and fermentation can be performed simultaneously. Such processes may be referred to as simultaneous saccharification fermentation (SSF). Combinations of chemical and enzymatic hydrolysis may also be performed in certain embodiments.

Chemical partial hydrolysis can be performed under any suitable conditions. In certain embodiments, chemical partial hydrolysis employs an acid. In specific embodiments, the acid comprises, consists essentially of or consists of sulphuric acid or nitric acid or hydrochloric acid. Concentrated acids may be employed in suitable amounts as would be readily determined by one skilled in the art in order to achieve the desired levels of hydrolysis. In specific embodiments, the acid is employed at a concentration of around 1-10% acid, or more specifically 0.5-5% acid. Suitable acid hydrolysis conditions are described herein.

Partial hydrolysis may also be performed at a suitable temperature. A temperature elevated over room (or ambient) temperature may facilitate the hydrolysis process. Thus, in specific embodiments (chemical) partial hydrolysis is performed at a temperature between around 50 and 200 degrees celcius (°C), more specifically between around 100 and 150°C and even more specifically between around 120 and 140°C. Thus, an acid may be used to hydrolyse the fermentation feedstock or the non-ethanol by-product of a fermentation process performed on a fermentation feedstock at any of these temperatures.

Similarly, the partial hydrolysis is performed for an appropriate period of time to ensure the desired level of conversion of non starch polysaccharides to soluble oligomers and monomers. In certain embodiments, chemical partial hydrolysis is performed for a period of between around 10 minutes and 5 hours, more specifically between around 20 minutes and 3 hours, or even more specifically between around 30 and 120 minutes. Thus, an acid may be used to hydrolyse the fermentation feedstock or the non-ethanol by-product of a fermentation process performed on a fermentation feedstock over any of these time periods.

Enzyme hydrolysis may be performed instead of, or together with, chemical hydrolysis. If both approaches are combined they may be performed simultaneously, sequentially or separately. Temperature, time and concentration conditions may need to be adjusted accordingly depending upon the approach taken, as would be appreciated by a skilled person. For example, enzymes may not perform efficiently at low temperatures and may be (irreversibly) denatured at higher temperatures. The type of enzyme employed will depend upon the nature of the fermentation feedstock, or the non-ethanol by-product of a fermentation process performed on a fermentation feedstock. Typically, glycosidase enzymes are employed. In specific embodiments, enzyme hydrolysis is performed using a hemi-cellulase and/or a cellulase. Specific examples of such enzymes include glycan hydrolase, E.C.3.2.1 and/or cellulases such as endo beta-glucanases and beta-glucosidase.

In agreement with the source fermentation feedstock, the non-starch polysaccharides may comprise, consist essentially of or consist of hemicellulose. In specific embodiments, the non starch polysaccharides comprise at least around 10%, 20%, 30%, 40%, 50%, 60%, 70%., 80%, 90% hemicellulose. Similarly, in specific embodiments, the non starch polysaccharides comprise at least around 10%, 20%, 30%, 40%, 50%, 60%, 70%., 80%, 90% cellulose. Cellulose and hemicellulose may make up the total of the non starch polysaccharides in certain embodiments. Other polysaccharides may be present depending upon the source of the feedstock, such as pectins, glucans, gums and inulin.

As described in further detail herein, the processes of the invention may be specifically adapted to permit fermentation of pentose sugars. Thus, in certain embodiments, partial hydrolysis produces soluble oligomers and monomers which comprise, consist essentially of or consist of pentose sugars. The preferred microorganisms of the invention can ferment both pentose and hexose sugars and soluble oligomers and monomers will typically comprise both hexose and pentose sugars. In specific embodiments, the pentose sugars comprise, consist essentially of or consist of xylose and/or arabinose. Soluble oligomers may include disaccharides such as cellobiose

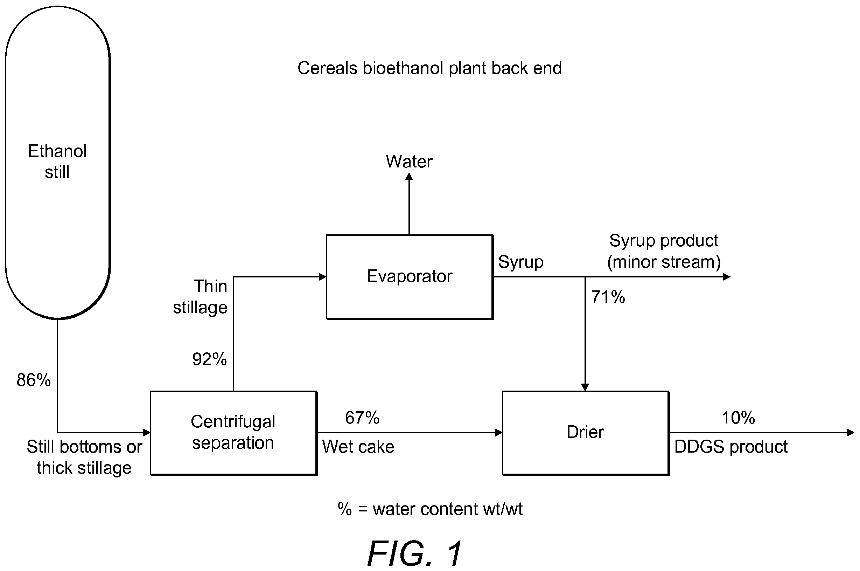

As discussed above, fermentation of a fermentation feedstock produces ethanol and a non-ethanol by-product. This is shown schematically in figure 1. Partial hydrolysis of the non-ethanol by-product of fermentation may be performed at any stage after fermentation has taken place. Thus, as defined in the claims, in step a) the non-ethanol by-product of a fermentation process performed on a fermentation feedstock is, or is derived from, the still bottoms or stillage from ethanol production. In specific embodiments, partial hydrolysis may be performed on the stillage, which may comprise, consist essentially of or consist of the thin stillage and/or thick stillage.

As described above, following partial hydrolysis of the non-ethanol by-product of a fermentation process performed on a fermentation feedstock, the soluble oligomers and monomers in the partially hydrolysed non-ethanol by-product (of fermentation) are fermented to produce ethanol. Suitable fermentation procedures are well known in the art and readily applied to optimise ethanol production. Fermentation is typically anaerobic but may be carried out under partially aerobic conditions in certain embodiments, as discussed herein. As discussed above, the fermentation may include fermentation of pentose sugars. Thus, in specific embodiments, fermentation is performed using a microorganism capable of fermenting pentose sugars, which may be a bacterium or a yeast, for example. In more specific embodiments, fermentation is performed using a thermophilic microorganism, in particular a thermophilic bacterium capable of fermenting pentose sugars. The thermopilic bacterium may lack lactate dehydrogenase activity. Lactate deficient mutants have previously been shown to be capable of producing increased ethanol yields. Suitable techniques for inactivating the *ldh* gene (encoding lactate dehydrogenase) are described for example in WO 2007/110608, WO 02/29030 and WO 2006/117536. Thus, the *ldh* gene may be inactivated through an insertion, deletion or substitution mutation. Lactate production stops and the excess pyruvate diverts mainly into the growth-linked pyruvate formate lyase (PFL) pathway. Thus, the thermophilic bacteria typically express pyruvate formate lyase. However, at very high sugar concentrations and/or at acid pH, the PFL pathway flux decreases and the excess pyruvate then overflows into an anaerobic pyruvate dehydrogenate (PDH) pathway, which ultimately yields only ethanol and CO₂. Therefore the preferred conditions to obtain high ethanol yields may be those that reduce flux through the PFL pathway and increase flux via the PDH pathway (Hartley, B.S. and Shama, G. Proc. Roy. Soc. Lond. 321, 555-568 (1987)). Unfortunately, under such conditions the cells may experience metabolic stress, with reduced ATP production, and a potential imbalance in NAD/NADH and CoA/acetyl CoA ratios

In order to address this possible issue of redox imbalance, especially under conditions of high sugar levels (produced by the partial hydrolysis), in certain embodiments, the thermophilic bacterium expresses a heterologous NAD-linked (or NAD-dependent) formate dehydrogenase (FDH). Many genes encoding NAD-linked FDH are known in the art (see for example Nanba et al (Biosci. Biotechnol. Biochem. 67(10), 2145-2153 (2003)) and may be employed to transform a suitable thermophilic bacterium. Thus, the thermophilic bacterium may be transformed with an *fdh* gene, in particular an *fdh*1 gene. The thermophilic bacterium may incorporate a gene encoding a thermostable NAD-linked formate dehydrogenase in certain embodiments. In other embodiments, the thermophilic bacterium may be transformed with a gene whose nucleotide sequence has been codon optimised to facilitate expression by the thermophilic bacterium. Production of such a thermostable NAD-linked formate dehydrogenase is described in detail in WO 2007/110608. In a specific embodiment, the gene encoding an NAD-linked formate dehydrogenase comprises, consists essentially of or consists of the nucleotide sequence set forth as SED ID NO: 1. In a further embodiment, the thermophilic bacterium incorporates a codon optimised (for expression in (Geo)Bacillus) gene encoding a thermostable NAD-linked formate dehydrogenase comprising, consisting essentially of or consisting of the nucleotide sequence set forth as SEQ ID NO:2. This sequence includes, in addition to the basic thermostable NAD-linked dehydrogenase sequence, promoter and terminator regions and also suitable restriction sites, such as, *Xba*1 sites to facilitate cloning of the gene into a suitable DNA construct.

In a still further embodiment the gene encoding an NAD-linked formate dehydrogenase is the *fdh*1 gene. The *fdh*1 gene may be derived from any suitable source and is preferably codon optimised for expression in the relevant thermophilic bacterium.

The fermentation thus may utilise a synthetic NAD-linked formate dehydrogenase, designed for optimum gene expression due to the use of the codon preferences of the appropriate thermophilic bacterium. The synthetic gene may contain engineered restriction sites to assist insertion into the lactate dehydrogenase gene. Thereby inactivation of the *ldh* gene and expression of the *fdh* gene are achieved in a single operation. In specific embodiments, the thermostable NAD-linked formate dehydrogenase remains functional at or above a temperature of 60 °C. The thermostable enzyme may be encoded by a nucleotide sequence which has been codon optimised for expression in a thermophilic bacterium. The formate dehydrogenase may comprise, consist essentially of or consist of the amino acid sequence set forth as SEQ ID NO: 3, as described in WO 2007/110608. Here, a specific thermostable NAD-linked formate dehydrogenase was designed based upon the amino acid sequence of the *Pseudomonas* sp 101 formate dehydrogenase (SEQ ID NO:3) and through use of optimised codons for *Geobacillus thermoglucosidasius.* The skilled person will appreciate that derivatives of this basic sequence will retain functionality. For example, conservative and semi-conservative substitutions may result in thermostable NAD-linked formate dehydrogenases and these derivatives are intended to fall within the scope of the invention provided they retain effective catalytic activity and thermostability such that they are useful in ethanol production using thermophilic bacteria. Similarly, minor deletions and/or additions of amino acids may produce derivatives retaining appropriate functionality.

In certain embodiments, in order to address the possible issue of redox imbalance, the fermentation process may be carried out under partially aerobic conditions. As the PDH pathway also operates under aerobic conditions where its operation leads to mainly cell mass production, the metabolic stress mentioned above can be relieved by partial sparging of air, generally performed at an optimum air sparging rate. By optimum air sparging rate is meant a sparging rate that is (just) sufficient to relieve the metabolic stress by allowing a low level of flux through the aerobic PDH pathway. This low level of flux does not, however, allow any significant decrease in the anaerobiosis and hence in the anaerobic PDH flux of the process. This means that there should be no significant decrease in ethanol production levels. Furthermore, because of the severe sensitivity of the PFL pathway towards air, this air sparging may have the additional benefit of reducing the flux through the PFL pathway and further increasing the flux through the anaerobic PDH pathway, but without putting the microorganism under metabolic stress. Suitable air sparging rates can readily be determined by one skilled in the art by investigating in the context of any particular fermentation process which rates result in optimal ethanol production levels and/or which minimise production of formate and acetate. Air sparging may be periodic or continuous and the rate can be adjusted accordingly. The skilled person would also realise that equivalent techniques to sparging could be employed to expose the fermentation to a limited amount of air, to achieve the desired effect. Also, the skilled person would realise that air could be replaced by an oxygen source if desired and the rates altered (reduced) accordingly. Thus, in a further aspect, the invention relates to ethanol production from C5 and C6 sugars under optimum air sparging levels. Optimisation is achieved by monitoring the redox level at which lowest formate and acetate levels result from the fermentation, while the comparatively highest level of ethanol concentrations are achieved in the process.

Any suitable thermophilic bacterium may be employed in the methods of the invention. In specific embodiments, the thermophilic bacterium is in the family Bacillaceae, more particularly the thermophilic bacterium may be of the genus *Geobacilllus.* In specific embodiments, the *Geobacillus* comprises *Geobacillus thermoglucosidasius* or *Geobacillus stearothermophilus.,* in particular a strain of *Geobacillus thermoglucosidasius* or *Geobacillus stearothermophilus* transformed with a gene encoding an NAD-linked formate dehydrogenase.

Whilst thermophilic bacteria have low tolerance to ethanol, this can conveniently be overcome in the fermentation by regular or continuous removal of ethanol. This ensures that the ethanol concentration in the fermentation is kept below the ethanol tolerance of the thermophilic bacterium. Ethanol may be continuously and conveniently removed from the (high temperature) fermentation by evaporation or distillation, such as membrane and/or mild vacuum evaporation for example. Fermentation may be performed within a temperature range of around 40°C and 80°C in some embodiments, such as between around 50°C and 70°C.

In the methods of the invention ethanol is produced through fermentation of the products of partial hydrolysis. The invention is based upon this combination of features, resulting in an improved animal feed product derived from the non-ethanol by-product of the fermentation. In specific embodiments, recovery of the non-ethanol by-product of fermentation following the fermentation step which takes place after or during partial hydrolysis, comprises:
- the centrifugal separation of thin stillage and wet cake from the still bottoms or thick stillage
- evaporation of the thin stillage
- recombining of the syrup resulting from the evaporation with the wet cake
- drying the recombined material to produce a dry product

In alternative embodiments, recovery of the non-ethanol by-product of fermentation comprises drying of still bottoms or thick stillage to produce a dry product

The nature of the animal feed product is determined by the fermentation feedstock employed in the processes of the invention and the steps performed after fermentation. Thus, in certain embodiments, the animal feed product comprises distillers grain (DG), distillers dried grain (DDG), distillers solubles (DS), distillers dried grains with solubles (DDGS) and/or vinasse. The vinasse may be sugar beet vinasse.

As discussed herein, the methods of the invention produce an animal feed product with improved nutritional content by virtue of the reduction in the levels of anti-nutritives. More specifically, the release of reducing and/or pentose sugars through partial hydrolysis, followed by fermentation of these sugars to ethanol improves the quality of the animal feed product derived from the non-ethanol by-product of fermentation. Thus, in specific embodiments, the improved nutritional content (of the animal feed product) comprises one or more of decreased levels of pentose sugars, increased relative protein concentration, decreased relative fibre concentration, decreased levels of soluble oligomers and monomers and decreased levels of reducing sugars.

The invention therefore also relates to an animal feed product produced according to the methods of the invention. In some embodiments, the product comprises less than 10, 9, 8, 7, 6 or 5% by weight of hemicellulose or 5, 4, 3, 2 or 1% by weight of pentose sugars, such as xylose.

The invention will be further described with reference to the following non-limiting experimental examples:

### BRIEF DESCRIPTION OF THE DRAWINGS

Figure 1 is a schematic flow diagram of a cereals bioethanol plant back end.
Figure 2 is a chromatogram of untreated thin stillage.
Figure 3 is a chromatogram of thin stillage hydrolysed with nitric acid.
Figure 4 is an overlay of the chromatograms of untreated (dashed line) and nitric acid treated (solid line) thin stillage samples.
Figure 5 is a chromatogram of thin stillage treated with enzymes at pH 5.0 and a temperature of 50°C for 24 hours.
Figure 6 is an overlay of the chromatograms of untreated (dashed line) and enzyme treated (solid line) thin stillage samples.

### DESCRIPTION OF THE PREFERRED EMBODIMENTS OF THE INVENTION

### EXAMPLE 1 - Acid and Enzyme hydrolysis of Thin Stillage

### Introduction

Thin stillage was obtained from a bioethanol process (Ensus). Figure 1 shows the Ensus process and the point where thin stillage is produced in the process. Percentage water content (wt/wt) is shown for each step of the process. Thin stillage is the semi-solid residue stream of the ethanol process and it is obtained after removing wet cake from the residue. The thin stillage is expected to be starch and glucose free and the carbohydrates will mainly be cellulosic and hemicellulosic residues. It is also expected that its hydrolysis, under optimum conditions, will release most of the sugars from these materials.

### Materials and Methods

### Thin stillage

Thin stillage was made available by Ensus from their bioethanol plant (See the introduction section and Figure 1).

### Acid hydrolysis of thin stillage

In a 100 ml Duran bottle containing about 12.5 ml of thin stillage, 0.125 ml of concentrated nitric or 0.136 ml of sulphuric acid was added and hydrolysed (autoclaved) at 121°C for 30 minutes.

### Enzyme hydrolysis of thin stillage

In 250 ml conical flasks containing about 50 - 100 ml of thin stillage (adjusted to pH 4 or 5), different levels of enzyme(s) according to Table 1 below were added. The flasks were then incubated at 60 - 60C°C for various intervals. Samples were drawn from the flasks and analysed by HPLC.

**Table 1 - Enzymes used in hydrolysis of thin stillage. Enzyme concentration was calculated on the basis of the solid contents of thin stillage (TS) as 8% w/v.**

| Genencor Enzymes | Recommended concentrations (g/g dry weight) | Amount added/100 ml TS* |
|---|---|---|
| Accellerase Duet | 0.05-0.25 | 2 ml |
| Accellerase 1500 | 0.05-0.25 | 2 ml |
| Optimash BG | 0.025-0.05 | 0.72ml |
| Optimash TBG | 0.025-0.05 | 0.72ml |
| Accellerase XY | 0.005-0.05 | 0.4 ml |
| Accellerase XC | 0.0125-0.125 | 1.2 ml |

### Dried insoluble solids in thin stillage

Total solids in the thin stillage were calculated by centrifuging the thin stillage at 4000 rpm for 10 to 20 minutes, removing the supernatant and drying it at 65°C for 48 hours.

### Dried soluble + insoluble solids in thin stillage

Thin stillage was kept at 120°C until a constant weight was obtained (in about 8 minutes).

### Analysis of thin stillage

Hydrolysed and unhydrolysed thin stillage was doubly centrifuged at 14000 rpm for 5 minutes and then filtered through 0.2 micron filter and analysed through a Dionex HPLC machine fitted with Dionex CarboPAC PA1 column kept at ambient temperature and eluted with gradient mobile phase [100% A (50mM NaOH) for 20 minutes followed by 100% B (250mM NaOAc/250mM NaOH) for 10 minutes followed by 15 minutes column regeneration with 100% A. Total time is 45 minutes at the flow rate of 1 ml/min.

Alternatively, the samples were analysed using Shimadzu HPLC machine fitted with Bio-Rad Aminex-HPX-87H column kept at 65C temperature and eluted with 5 mM sulphuric acid for 25 minutes at the flow rate of 0.6 ml/min.

### Results

### Total solids in thin stillage

Total solids (from soluble + insoluble) in the thin stillage were found to be between 60 and 85 g/l when the thin stillage was dried at 120°C for 8 minutes and most of the thin stillage batches had around 80 g/l total solid contents. Total insoluble solids were found to be about 45 g/l when the solids were first separated from the residues and then the residue was dried at 65°C for 48 hours.

### Carbohydrate Release by Acid Hydrolysis of thin stillage

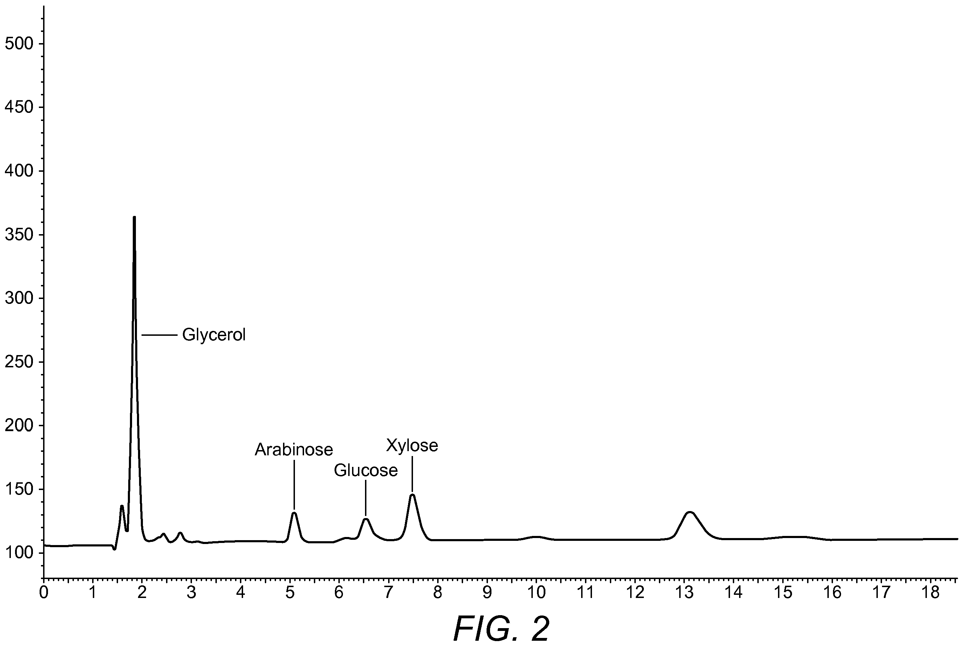

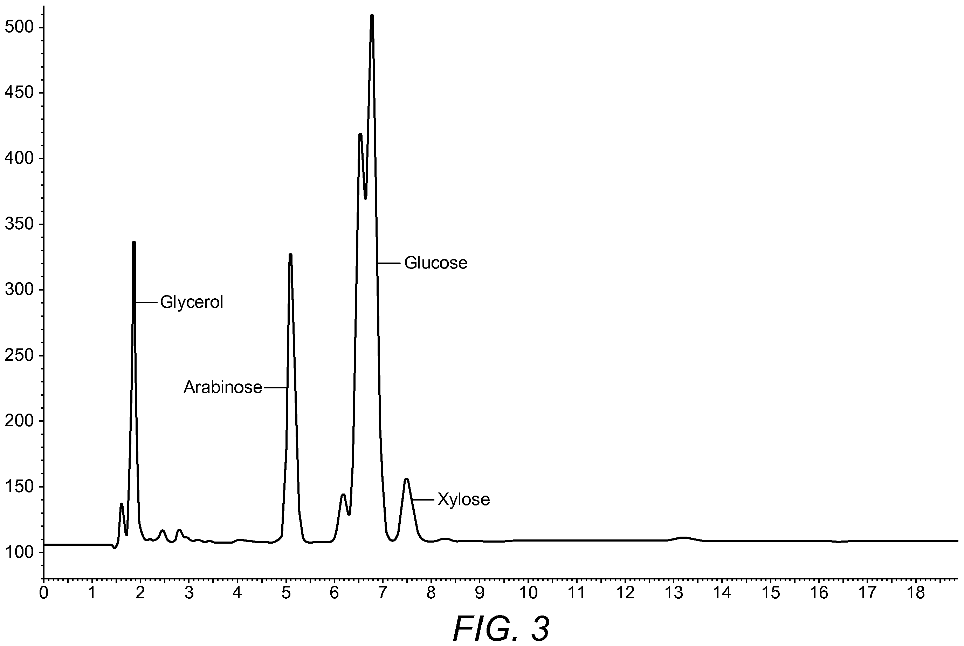

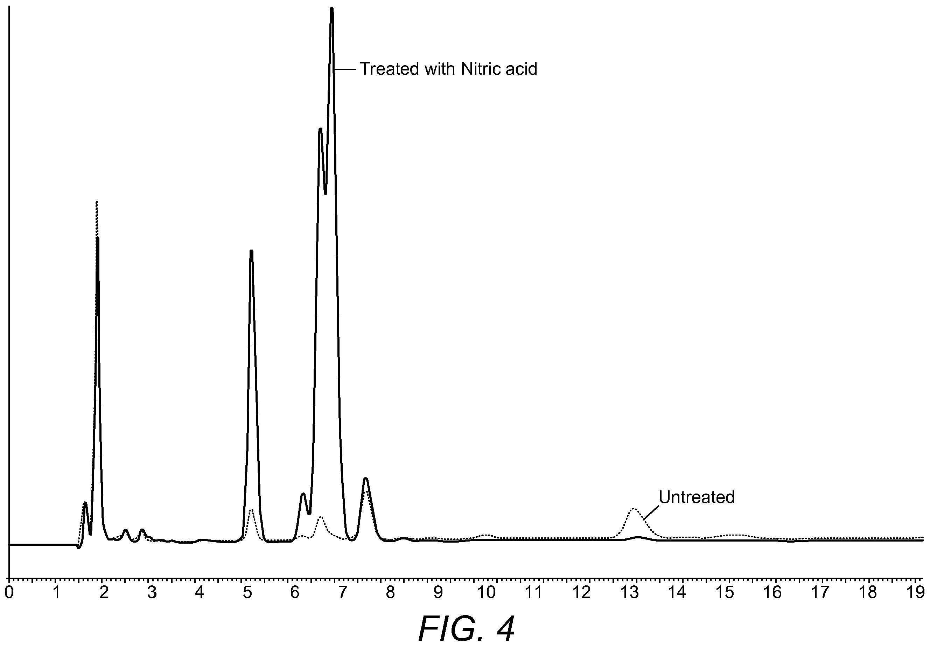

The results in Table 2 (and Figure 2) show that very small amount of sugars were present in the thin stillage (only 1.7 g/l of monomers sugars and 3 g/l of dimer sugars). Simple autoclaving released only a very small amount of monomer sugars (about 1.8 g/l). However, acid hydrolysis released a significant amount of sugars from the thin stillage. Hydrolysis of the stillage at 121°C for 30 minutes with 1% nitric acid increased the soluble monomer sugars from 1.7 g/l to about 26 g/l while reduced the dimer sugars from 3 g/l to less than 0.1 g/l (Figures 3 and 4). A similar reduction in the dimer levels was achieved with 1% sulphuric acid treatment while the increase in the monomer sugars to 22.5 g/l was marginally less than that achieved with nitric acid.

### Carbohydrate Release by Enzyme Hydrolysis of thin stillage

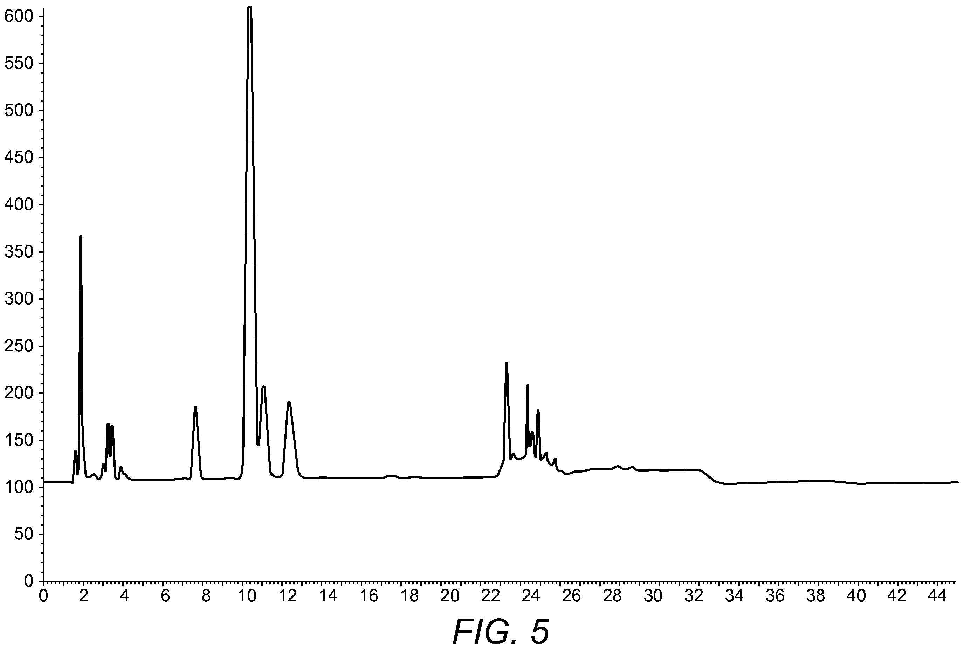

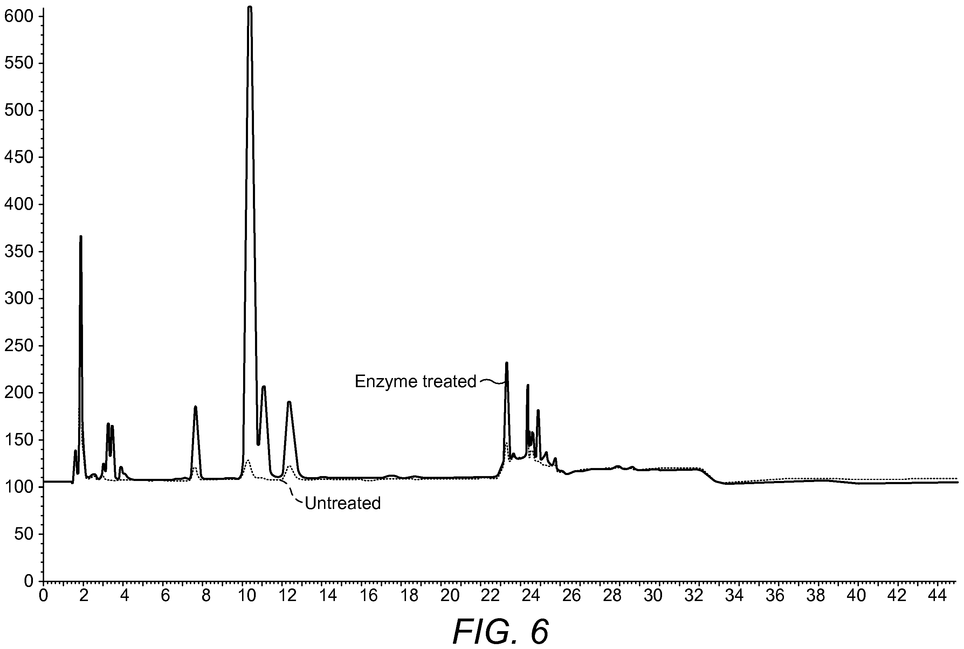

Qualitative results presented in Figures 5 and 6 clearly indicate that a significant amount of monomer sugars were released during the enzymatic hydrolysis. The quantitative results of the hydrolysis of thin stillage with different enzymes are shown in Table 3. Accellerase 1500, Accellerase Duet, Optimash GB and Optimash TBG release significant amounts of sugars and up to about 25 g/l of sugar could be released from the thin stillage. While Accellerase XC and Accellerase XY also release some sugars from the thn stillage.

### Conclusions

- The dried insoluble solid content of the thin stillage is about 45 g/l
- The dried solid content (soluble + insoluble) of the thin stillage is between 60 and 85 g/l
- About 21 g/l sugars with 1% sulphuric and about 23 g/l sugars with 1% nitric acid were released from the hydrolysis of the thin stillage.
- Enzymatic hydrolysis also released a significant amount of sugars from the thin stillage.

**Table 2: Carbohydrate levels (g/l) in thin stillage with and without treatment**

| **Treatment** | **Glucose** | **Xylose** | **Arabinose** | **Others** | **Total Mono.** |
|---|---|---|---|---|---|
| None | 0 | 0.73 | 1.01 | 3.01 | 1.74 |
| 121°C - 30 min | 0.29 | 2.14 | 1.07 | 3.2 | 3.50 |
| 121°C - 30 min with 1% HNO³ | 6.76 | 13.36 | 5.84 | 0.08 | 25.96 |
| 121°C - 30 min with 1% H₂S0₄ | 5.54 | 11.95 | 5.01 | 0.07 | 22.50 |

| | | | | | |
|---|---|---|---|---|---|
| Mono. = monomer sugars | | | | | |

The solids in thin stillage were 45 g/l.

**Table 3: Carbohydrate levels (g/l) in thin stillage released with different enzymes (Genencor) at 60°C and pH 5 after 24 hours.**

| **Enzymes** | **Amount Added/100 mL TS** | **Arabinose (g/L)** | **Glucose (g/L)** | **Xylose (g/L)** | **Total sugars (g/L)** |
|---|---|---|---|---|---|
| Accellerase Duet | 0.125mL | 0.69 | 12.11 | 4.39 | 23.19 |
| Accellerase 1500 | 2mL | 5.5 | 17.9 | 2 | 25.5 |
| Optimash BG* | 0.72mL | 1.76 | 16.10 | 2.25 | 20.11 |
| Optimash TBG* | 0.72mL | 2.45 | 19.26 | 3.42 | 25.12 |
| Accellerase XY | 0.4mL | 6 | 10.9 | 0 | 17.3 |
| Accellerase XC** | 1.2mL | 3.7 | 3.64 | 0.48 | 11.8 |

| | | | | | |
|---|---|---|---|---|---|
| *, Hydrolysis was performed at pH 4 (instead of pH 5) and at 50C (instead of 60C) **, Hydrolysis was carried out for 6 hours (instead of 24 hours). | | | | | |

The present invention is not to be limited in scope by the specific embodiments described herein. Indeed, various modifications of the invention in addition to those described herein will become apparent to those skilled in the art from the foregoing description and accompanying figures. Such modifications are intended to fall within the scope of the appended claims. Moreover, all embodiments described herein are considered to be broadly applicable and combinable with any and all other consistent embodiments, as appropriate.

## Claims

1. A method for production of an animal feed product, the method comprising, as part of an existing bioethanol production process:
a) partial hydrolysis of the non-ethanol by-product of a fermentation process performed on a fermentation feedstock, which partial hydrolysis converts non starch polysaccharides to soluble oligomers and monomers, wherein the non-ethanol by-product of a fermentation process performed on a fermentation feedstock is, or is derived from, the still bottoms or stillage from ethanol production
b) fermentation of the soluble oligomers and monomers in the partially hydrolysed non-ethanol by-product to produce additional ethanol compared to that produced by the existing bioethanol production process
c) recovery of the non-ethanol by-product from the fermentation of step b) to produce an animal feed product with improved nutritional content.

2. The method of claim 1 wherein:
a) the fermentation feedstock comprises a hemi-cellulose containing plant material and/or,
b) wherein the partial hydrolysis comprises up to around 75% hydrolysis of the non starch polysaccharides and/or,
c) wherein the non starch polysaccharides comprise at least 50% hemicellulose and/or,
d) wherein the soluble oligomers and monomers comprise pentose sugars optionally wherein the pentose sugars comprise xylose and/or arabinose.

3. The method of any preceding claim wherein:
a) the stillage is thin stillage or thick stillage and/or,
b) the animal feed product comprises distillers grain, distillers dried grain, distillers solubles, distillers dried grains with solubles or vinasse.

4. The method of any preceding claim wherein the partial hydrolysis is performed chemically and/or enzymatically optionally wherein the chemical partial hydrolysis employs an acid optionally wherein the acid is sulphuric acid, nitric acid or hydrochloric acid.

5. The method of claim 4 wherein:
a) the acid is employed at a concentration of around 0.5-5% acid and/or,
b)wherein the acid is employed at a temperature of between around 100 and 150 deg C and/or,
c) wherein the acid is employed for a period of between around 20 and 120 minutes.

6. The method of claim 5 wherein the enzyme or enzymes comprise a hemi-cellulase and/ or a cellulase.

7. The method of any preceding claim wherein the fermentation of the soluble oligomers and monomers in step b) is carried out under partially aerobic conditions, optionally wherein partially aerobic conditions are achieved by air sparging.

8. The method of any preceding claim wherein the fermentation of the soluble oligomers and monomers in step b) is carried out by a thermophilic bacterium, optionally wherein:
a) the thermophilic bacterium lacks lactate dehydrogenase activity and/or,
b) the thermophilic bacterium expresses a heterologous NAD-linked formate dehydrogenase and/or,
c) the thermophilic bacterium is of the genus Geobacilllus optionally wherein the Geobacillus comprises *Geobacillus thermoglucosidasius* or *Geobacillus stearothermophilus.*

9. The method of any preceding claim wherein the ethanol produced in step b) is removed by evaporation or distillation.

10. The method of any preceding claim wherein recovery of the non-ethanol by-product of fermentation in step c) comprises:
(a) centrifugal separation of thin stillage and wet cake from the still bottoms or thick stillage
(b) evaporation of the thin stillage
(c) recombining of the syrup resulting from the evaporation with the wet cake
(d) drying the recombined material to produce a dry product

11. The method of any preceding claim wherein recovery of the non-ethanol by-product of fermentation in step c) comprises drying of still bottoms or thick stillage to produce a dry product.

12. The method of any preceding claim wherein the improved nutritional content comprises one or more of decreased levels of pentose sugars, increased protein concentration, decreased fibre concentration, decreased levels of soluble oligomers and monomers, decreased levels of reducing sugars.

13. An animal feed product produced according to the method of any one of claims 1 to 12.

14. The animal feed product according to claim 13 wherein the product comprises less than 10% by weight of hemicellulose or 5% by weight of pentose sugars.

## Patentansprüche

1. Verfahren zur Herstellung eines Tierfutterprodukts, wobei das Verfahren als Teil eines existierenden Bioethanol-Herstellungsprozesses umfasst:
a) partielle Hydrolyse des Nicht-Ethanol-Nebenprodukts eines Fermentationsprozesses, durchgeführt an einem Fermentationsausgangsmaterial, wobei die partielle Hydrolyse Nicht-Stärke-Polysaccharide in lösliche Oligomere und Monomere umwandelt, wobei das Nicht-Ethanol-Nebenprodukt eines Fermentationsprozesses, durchgeführt an einem Fermentationsausgangsmaterial, die Destillationsrückstände oder die Schlempe von einer Ethanol-Herstellung ist oder davon abgeleitet ist
b) Fermentation der löslichen Oligomere und Monomere in dem partiell hydrolysierten Nicht-Ethanol-Nebenprodukt um zusätzliches Ethanol herzustellen, verglichen mit dem, das durch den existierenden Bioethanol-Herstellungsprozess hergestellt wird
c) Rückgewinnung des Nicht-Ethanol-Nebenprodukts aus der Fermentation von Schritt b), um ein Tierfutterprodukt mit verbessertem Nährstoffgehalt herzustellen.

2. Verfahren nach Anspruch 1, wobei:
a) das Fermentationsausgangsmaterial eine Hemicellulose umfasst, die Pflanzenmaterial enthält und/oder,
b) wobei die partielle Hydrolyse bis zu ungefähr 75% Hydrolyse der Nicht-Stärke-Polysaccharide umfasst und/oder,
c) wobei die Nicht-Stärke-Polysaccharide wenigstens 50% Hemicellulose umfassen und/oder,
d) wobei die löslichen Oligomere und Monomere Pentosezucker umfassen, wobei die Pentosezucker gegebenenfalls Xylose und/oder Arabinose umfassen.

3. Verfahren nach einem der vorhergehenden Ansprüche, wobei:
a) die Schlempe dünne Schlempe oder dicke Schlempe ist und/oder,
b) das Tierfutterprodukt Getreideschlempe (distillers grain), Trockenschlempe (distillers dried grain), Dünnschlempe (distillers solubles), Trockenschlempe mit löslichen Stoffen (distillers died grains with solubles) oder Vinasse umfasst.

4. Verfahren nach einem der vorhergehenden Ansprüche, wobei die partielle Hydrolyse chemisch und/oder enzymatisch durchgeführt wird, wobei gegebenenfalls die chemische, partielle Hydrolyse eine Säure einsetzt, wobei gegebenenfalls die Säure Schwefelsäure, Salpetersäure oder Salzsäure ist.

5. Verfahren nach Anspruch 4, wobei:
a) die Säure in einer Konzentration von ungefähr 0,5 - 5 % Säure eingesetzt wird und/oder,
b) wobei die Säure bei einer Temperatur von zwischen ungefähr 100 und 150 Grad C eingesetzt wird und/oder,
c) wobei die Säure für einen Zeitraum von zwischen ungefähr 20 und 120 Minuten eingesetzt wird.

6. Verfahren nach Anspruch 5, wobei das Enzym oder die Enzyme eine Hemicellulase und/oder eine Cellulase umfasst / umfassen.

7. Verfahren nach einem der vorhergehenden Ansprüche, wobei die Fermentation der löslichen Oligomere und Monomere in Schritt b) unter partiell aeroben Bedingungen durchgeführt wird, wobei gegebenenfalls partiell aerobe Bedingungen durch Luft-Einblasen erreicht werden.

8. Verfahren nach einem der vorhergehenden Ansprüche, wobei die Fermentation der löslichen Oligomere und Monomere in Schritt b) durch ein thermophiles Bakterium durchgeführt wird, wobei gegebenenfalls:
a) dem thermophilen Bakterium Laktat-Dehydrogenase-Aktivität fehlt und/oder,
b) das thermophile Bakterium eine heterologe NAD-verknüpfte Formiat-Dehydrogenase exprimiert und/oder
c) das thermophile Bakterium von der Gattung Geobacillus ist, wobei gegebenenfalls das Geobacillus *Geobacillus thermoglucosidasius* oder *Geobacillus stearothermophilus* umfasst.

9. Verfahren nach einem der vorhergehenden Ansprüche, wobei das Ethanol, das in Schritt b) hergestellt wird, durch Verdampfung oder Destillation entfernt wird.

10. Verfahren nach einem der vorhergehenden Ansprüche, wobei die Rückgewinnung des Nicht-Ethanol-Nebenprodukts der Fermentation in Schritt c) umfasst:
(a) zentrifugale Trennung von dünner Schlempe und feuchtem Kuchen von Destillationsrückständen oder dicker Schlempe
(b) Verdampfung der dünnen Schlempe
(c) Wiedervereinigen des Sirups, das aus der Verdampfung entsteht, mit dem feuchten Kuchen
(d) Trocknen des wiedervereinigten Materials um ein trockenes Produkt herzustellen.

11. Verfahren nach einem der vorhergehenden Ansprüche, wobei die Rückgewinnung des Nicht-Ethanol-Nebenprodukts der Fermentation in Schritt c) ein Trocknen von Destillationsrückständen oder dicker Schlempe umfasst, um ein trockenes Produkt herzustellen.

12. Verfahren nach einem der vorhergehenden Ansprüche, wobei der verbesserte Nährstoffgehalt eines oder mehrere umfasst von verringerte Level an Pentosezuckern, erhöhte Proteinkonzentration, verringerte Ballaststoffkonzentration, verringerte Levels an löslichen Oligomeren und Monomeren, verringerte Levels an reduzierenden Zuckern.

13. Tierfutterprodukt, hergestellt nach dem Verfahren nach einem der Ansprüche 1 bis 12.

14. Tierfutterprodukt nach Anspruch 13, wobei das Produkt weniger als 10 Gewichts-% an Hemicellulose oder 5 Gewichts-% an Pentosezuckern umfasst.

## Revendications

1. Procédé de production d'un produit alimentaire pour animaux, le procédé comprenant, dans le cadre d'un procédé de production de bioéthanol existant :
a) l'hydrolyse partielle du sous-produit non-éthanolique d'un procédé de fermentation effectué sur une charge d'alimentation de fermentation, laquelle hydrolyse partielle convertit les polysaccharides non-amylacés en oligomères et monomères solubles, où le sous-produit non-éthanolique d'un procédé de fermentation effectué sur une charge d'alimentation de fermentation est, ou est dérivé, des culots de distillation ou des résidus de distillation provenant de la production d'éthanol
b) la fermentation des oligomères et des monomères solubles dans le sous-produit non-éthanolique partiellement hydrolysé pour produire de l'éthanol supplémentaire par rapport à celui produit par le procédé de production de bioéthanol existant
c) la récupération du sous-produit non-éthanolique de la fermentation de l'étape b) pour produire un produit alimentaire pour animaux à valeur nutritive améliorée.

2. Procédé de la revendication 1, dans lequel :
a) la charge d'alimentation de fermentation comprend une matière végétale contenant de l'hémicellulose et/ou,
b) dans lequel l'hydrolyse partielle comprend jusqu'à environ 75% d'hydrolyse des polysaccharides non-amylacés et/ou,
c) dans lequel les polysaccharides non-amylacés comprennent au moins 50% d'hémicellulose et/ou,
d) dans lequel les oligomères et les monomères solubles comprennent des sucres pentoses, facultativement où les sucres pentoses comprennent du xylose et/ou de l'arabinose.

3. Procédé de l'une des revendications précédentes dans lequel :
a) les résidus de distillation sont des résidus solubles de distillation ou des résidus de distillation épais et/ou,
b) le produit alimentaire pour animaux comprend des drêches de distillerie, des drêches sèches de distillerie, des solubles de distillerie, des drêches sèches de distillerie avec solubles ou de la vinasse.

4. Procédé de l'une des revendications précédentes, dans lequel l'hydrolyse partielle est effectuée chimiquement et/ou par voie enzymatique, facultativement où l'hydrolyse partielle chimique utilise un acide, facultativement où l'acide est l'acide sulfurique, l'acide nitrique ou l'acide chlorhydrique.

5. Procédé de la revendication 4, dans lequel :
a) l'acide est utilisé à une concentration d'environ 0,5 à 5% d'acide et/ou,
b) dans lequel l'acide est utilisé à une température comprise entre environ 100 et 150°C et/ou,
c) dans lequel l'acide est utilisé pendant une période comprise entre environ 20 et 120 minutes.

6. Procédé de la revendication 5, dans lequel l'enzyme ou les enzymes comprend/comprennent une hémicellulase et/ou une cellulase.

7. Procédé de l'une des revendications précédentes, dans lequel la fermentation des oligomères et des monomères solubles dans l'étape b) est réalisée dans des conditions partiellement aérobies, facultativement où les conditions partiellement aérobies sont obtenues par barbotage à l'air.

8. Procédé de l'une des revendications précédentes, dans lequel la fermentation des oligomères et des monomères solubles dans l'étape b) est réalisée par une bactérie thermophile, facultativement où :
a) la bactérie thermophile est dépourvue d'activité lactate déshydrogénase et/ou,
b) la bactérie thermophile exprime une formiate déshydrogénase hétérologue liée à NAD et/ou,
c) la bactérie thermophile est du genre Geobacilllus, facultativement où Geobacillus comprend *Geobacillus thermoglucosidasius ou Geobacillus stearothermophilus.*

9. Procédé de l'une des revendications précédentes, dans lequel l'éthanol produit dans l'étape b) est éliminé par évaporation ou distillation.

10. Procédé de l'une des revendications précédentes, dans lequel la récupération du sous-produit non-éthanolique de fermentation dans l'étape c) comprend :
a) la séparation centrifuge des résidus solubles de distillation et d'un gâteau humide des culots de distillation ou des résidus de distillation épais
(b) l'évaporation des résidus solubles de distillation
(c) la recombinaison du sirop résultant de l'évaporation avec le gâteau humide
(d) le séchage du matériau recombiné pour produire un produit sec.

11. Procédé de l'une des revendications précédentes, dans lequel la récupération du sous-produit non-éthanolique de fermentation dans l'étape c) comprend le séchage des culots de distillation ou des résidus de distillation épais pour produire un produit sec.

12. Procédé de l'une des revendications précédentes, dans lequel la valeur nutritive améliorée comprend un(e) ou plusieurs parmi des niveaux réduits de sucres pentoses, une concentration en protéine accrue, une concentration en fibres réduite, des niveau réduits d'oligomères et de monomères solubles, des niveaux réduits de sucres réducteurs.

13. Produit alimentaire pour animaux produit selon le procédé de l'une quelconque des revendications 1 à 12.

14. Produit alimentaire pour animaux selon la revendication 13, dans lequel le produit comprend moins de 10% en poids d'hémicellulose ou 5% en poids de sucres pentoses.
